(19) Europäisches Patentamt
European Patent Office
Office européen des brevets

(11) Publication number: **0 286 735 B1**

# (12) EUROPEAN PATENT SPECIFICATION

(45) Date of publication of patent specification: **29.05.91** (51) Int. Cl.5: **C07D 307/88**, A01N 43/12

(21) Application number: **87200698.6**

(22) Date of filing: **14.04.87**

## (54) Diphenyl ether derivatives.

(43) Date of publication of application:
**19.10.88 Bulletin 88/42**

(45) Publication of the grant of the patent:
**29.05.91 Bulletin 91/22**

(84) Designated Contracting States:
**AT BE CH DE ES FR GB GR IT LI LU NL SE**

(56) References cited:
**JP-A-62 061 973**

**CHEMICAL ABSTRACTS, vol. 80, no. 11, 18th March 1974, page 341, abstract no. 59757g, Columbus, Ohio, US; M. LACOVA: "Phthalides and 1,3-indandiones. LIII. Phthalides and 1,3-indandiones from (arylthio)acetic and aryloxyacetic acids", & CHEM. ZVESTI 1973, 27(4), 525-35**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Clark, Michael Thomas**
**Torcross Wises Lane Borden**
**Sittingbourne Kent(GB)**
Inventor: **Gilmore, Ian James**
**32 Waterloo Road**
**Sittingbourne Kent(GB)**
Inventor: **Jury, Donald Robert**
**101 Gadby Road**
**Sittingbourne Kent(GB)**

(74) Representative: **Bennett, David Arthur Horder et al**
**4, York Road**
**London SE1 7NA(GB)**

EP 0 286 735 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to certain diphenyl ether derivatives, the preparation of such derivatives, herbicidal compositions containing them and their use in combating undesired plant growth.

From European patent specification No. 145,078 it is known that a herbicidal activity is shown by diphenyl ether derivatives having general formula I

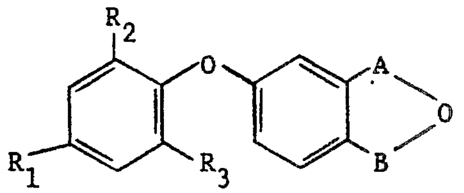

I

in which $R_1$ represents a hydrogen or halogen atom, or an alkyl or haloalkyl group;

$R_2$ and $R_3$ which may be the same or different, each independently represents a hydrogen or halogen atom or an alkyl, haloalkyl, nitro or cyano group; and A and B may inter alia represent a divalent group of formula >C=O, >C=S or $>C(R^1)_2$, in which the separate groups $R^1$ can be selected from several monovalent radicals; and European patent specification No. 219,144 describes a specific group of compounds of formula I characterised by an alkylidene group of formula $>C(alkyl)_2$ at position A, which have a superior herbicidal activity and hydrolytic stability compared with compounds specifically disclosed in EP-A-0 145 078.

It has now surprisingly been found that diphenyl ether derivatives of formula I in which the group A represents certain ethylenical groups show interesting herbicidal activity, of a level which is not shared by other compounds very similar in structure.

Accordingly, the present invention relates to diphenyl ether derivatives having general formula I, in which $R_1$, $R_2$ and $R_3$ have the above meanings, and in which A an ethylenical group of formula $>C=C(H)-(R_4)$, wherein $R_4$ represents a hydrogen or an alkyl group; and B represents a carbonyl group.

When the compounds of this invention contain an alkyl group, this may be linear or branched and may contain up to 10, preferably up to 6 and especially up to 4 carbon atoms, suitable examples being methyl, ethyl and propyl.

Preferred compounds are those wherein $R_1$ represents a haloalkyl group, in particular a trifluoromethyl group, $R_2$ represents a halogen, in particular chlorine, atom and $R_3$ is hydrogen.

In certain embodiments of the invention $R_4$ represents an alkyl group, preferably a $C_{1-4}$ alkyl group, for example methyl or ethyl.

In other embodiments of the invention $R_4$ represents a hydrogen atom.

The invention further provides a process for the preparation of a phthalide derivative as defined above, which comprises reacting a phthalide of formula II

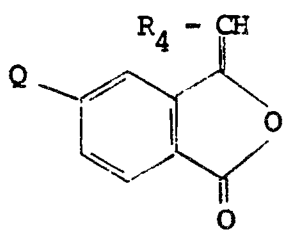

II

with a compound of formula III

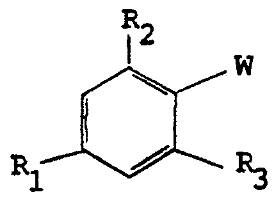

III

wherein $R_1$, $R_2$ and $R_3$ have the meanings given above for formula I, and one of Q and W represents a halogen atom or nitro group, and the other represents a group of formula -OM, wherein M represents a hydrogen or alkali metal atom. Conveniently the reaction is carried out by reacting a compound of formula II wherein Q is hydroxyl with an alkali metal hydroxide or hydride, preferably in a suitable solvent, thereby forming an alkali metal salt, and the alkali metal salt is reacted with the compound of formula III, wherein W represents a chlorine atom. The reaction of an alkali metal hydroxide with a compound of formula II is conveniently carried out in an alkanol solvent, e.g. ethanol The latter reaction and also the reaction of the alkali metal hydride with the compound of formula III and also the reaction of the alkali metal salt with the chloro-compound is preferably carried out in a suitable aprotic organic solvent, e.g. dimethyl sulphoxide, sulpholane, dimethyl formamide, or dimethyl acetamide. The latter reaction is suitably carried out at elevated temperature, e.g. above 25° C and in particular between 40° C and reflux, conveniently under reflux, and also under an inert atmosphere such as nitrogen.

In another suitable method to prepare the compounds of formula I a compound of formula IV is reacted

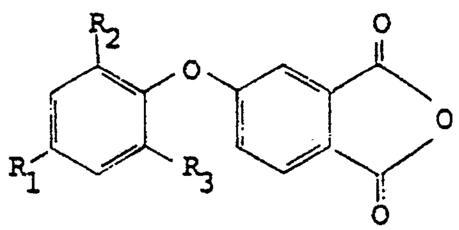

IV

with a compound of formula ($R_4$) $H_2C$-L, in which L is a suitable leaving group. Suitable leaving groups include carboxyl groups and their alkyl ester and alkali metal derivatives. The reaction is suitably carried out at elevated temperature, preferably between 150 and 400° C. Compounds wherein >C = C($R_4$)(H) represents >C = $CH_2$ may also be prepared by dehydration of pre-cursor compounds having a >CH-$CH_2OH$ substituent. So, it is e.g. possible to obtain a compound of formula V

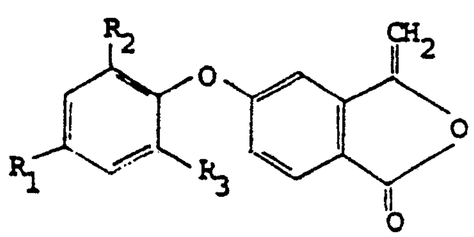

V

by dehydrating a compound of formula VI

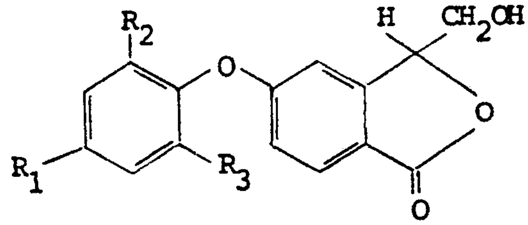

VI

The dehydration is suitably carried out at elevated temperature, e.g. from 50° C to reflux temperature, in the

presence of a carboxylic acid anhydride, e.g. acetic acid anhydride. Compounds of formula VI are conveniently prepared by halogenation e.g. bromination, of the corresponding methyl-substituted derivative, followed by hydration of the resulting halomethyl group. In some cases, the above reactions are carried out in a convenient solvent; in other cases one of the reactants is conveniently added in excess to provide a suitable reaction solvent.

This latter situation is convenient e.g. in the above-described hydration (in water) and dehydration (in carboxylic acid anhydride).

Another convenient process for preparing compounds of formula I comprises reacting a phthalide of formula VII

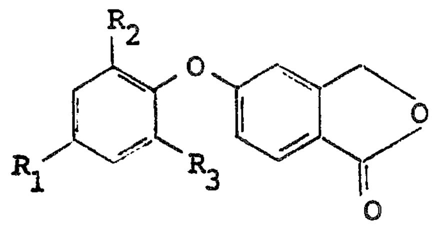

VII

wherein $R_1$, $R_2$ and $R_3$ have the above meanings with an acetal of the formula

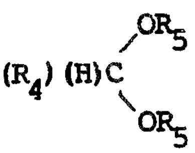

in which each $R_5$ individually represents an alkyl group, preferably containing from 1 to 4 carbon atoms. This reaction is conveniently carried out at elevated temperature, e.g. from 40°C to the reflux temperature of the reaction mixture, under nitrogen. Preferably, a catalyst is present. Suitable catalysts include the alkalimetal alkoxides e.g. potassium t-butoxide.

The starting materials described are known or may be prepared from known compounds by standard methods. For example compounds of formula III, VI (and its methyl precursor) and VII are disclosed in EP-A-145078. Compounds of formula IV are known (Chem. Abs. Vol. 73(19)no. 98664Z.) whilst compounds of formula II may be prepared from known compounds by modification of the phthalide part of the compound in analogous manner to that already described with respect to the other methods, that is, those applied to diphenyl ether phthalides.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier. It has been found that the compounds of the present invention have a surprisingly high level of herbicidal activity especially when compared with other compounds containing similar ethylenic groups.

The invention also provides the use of such a compound or composition according to the invention as a herbicide. Further, in accordance with the invention there is provided a method of combating undesired plant growth at a locus by treating the locus with a compound or composition according to the invention.

Application to the locus may be pre-emergence or post-emergence. The dosage of active ingredient used may, for example, be from 0.05 to 10kg/ha, preferably 0.1 to 4kg/ha.

A carrier in a composition according to the invention is any material with which the active ingredient is formulated to facilitate application to the locus to be treated which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for

example attapulgites and vermiculites; aluminium silicates, for example kaolinites, montmorillonites and micas; calcium carbonate, calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane.

Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols.

Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing -10% w of active ingredient. Granules are usually prepared to have a size between 10 and 100 BS mesh (1.676 -0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following Examples 1 and 2, whilst Example 3 to 8 describe related compounds outside the scope of the invention.

Example 1

Preparation of 5-(2-chloro-4-trifluoromethylphenoxy)-3-methylene-1-(3H)isobenzofuranone (1).

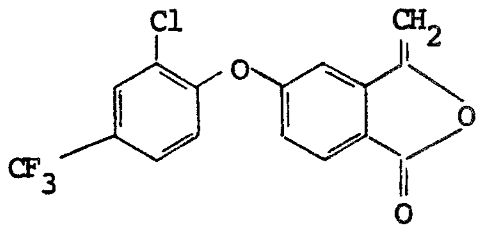

(1)

To 3.4g of 5-(2-chloro-4-trifluoromethylphenoxy)-3-methylphthalide in 50 ml carbon tetrachloride was added 2.0g of N-bromosuccimimide and a pellet of benzoylperoxide as a catalyst. The mixture was refluxed under bright light for 1½ hours. Then, the mixture was cooled, the solvent was removed the residue was added to 50ml of water and the resulting admixture was refluxed for 3 hours. Water was then removed, the residue was taken up in dry toluene and the toluene/water mixture was removed by azeotropic distillation to yield a dry solid. This solid was added to 50ml. acetic anhydride, and the mixture was refluxed overnight. The solvent was then removed, and a saturated bicarbonate solution was added to the residue. The aqueous phase was extracted with methylene chloride, the organic layer was washed with water, and dried. The organic solvent was removed and the residue was chromatographically purified over silica using methylene chloride as eluant, yielding 2.0g of (1), m.p. 92-94° C.

| | | | |
|---|---|---|---|
| Analysis | Calculated: | C 56.4; | H 2.4% |
| | Found : | C 55.8; | H 2.6% |

Examples 2 and 3

Preparation of 5- and 6-(2-chloro-4-trifluoromethyl-phenoxy)-3-methylmethylene-1-(3H)isobenzofuranone (2 and 3)

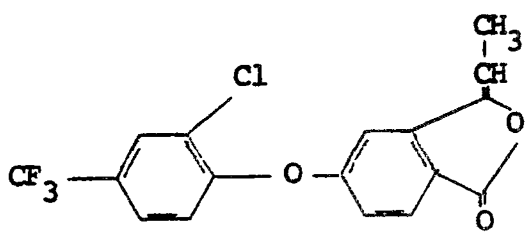

Example 2

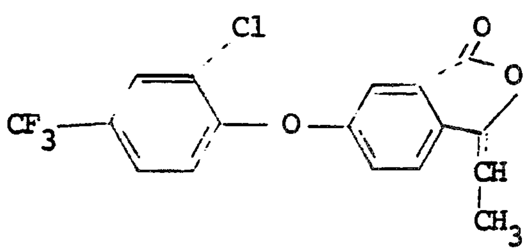

Example 3

5-(2-Chloro-4-trifluoromethylphenoxy)phthalic anhydride (34.25g) and sodium propionate (9.6g) in propionic anhydride (100ml) were stirred and heated in an oil bath at 170° C for 4 hours. Most of the anhydride was removed in a rotary evaporator and the solid residue was treated with toluene and water. The organic layer was separated, washed with demineralised water and evaporated to dryness. The residual oil was split into two equal portions. One portion was chromatographed on silica gel using dichloromethane as eluant, yielding 4.35g of (2) and 1.25g of (2)..
Recrystallization from toluene was effected. Example 2 - mp 121-123° C; Example 3 - mp 144-145.4° C.

| | | | | |
|---|---|---|---|---|
| Analysis: | Calculated | : | C 57.5; | H 2.8% |
| | Found (2) | : | C 57.5; | H 2.8% |
| | Found (3) | : | C 57.3; | H 2.9% |

Example 4 and 5

Preparation of 6- and 5-(2-chloro-4-trifluoromethyl-phenoxy)-3-(2-methoxyphenylmethylene)-1-(3H)-isobenzofuranone (4 and 5)

In an oil bath a mixture comprising 6.8g of 5-(2-chloro-4-trifluoromethylphenoxy)phthalic anhydride, 3.65g of o-methoxyphenylacetic acid and 4.9g of sodium acetate, was heated at 230-240° C for 2 hours. The mixture was cooled and subjected to extraction with boiling ethanol. The hot ethanol solution was filtered and allowed to cool off yielding a yellow precipitate. This precipitate was collected and dissolved in methylene chloride. Purification by HPLC using 20% THF in hexane as eluant gave 1.15g of (4), m.p. 152° C, and 1.35g of (5), m.p. 147° C

| | | | | |
|---|---|---|---|---|
| Analysis | Calculated | : | C 61.8; | H 3.1% |
| | Found (4) | : | C 62.0; | H 3.5% |
| | Found (5) | : | C 61.1; | H 3.2% |

Examples 6 and 7

Preparation of 6- and 5-(2-chloro-4-trifluoromethyl-phenoxy)3-phenylmethylene-1-(3H)isobenzofuranone (6 and 7)

In a similar way as described in Examples 4 and 5, 6.8g of 5-(2-chloro-4-trifluoromethylphenoxy)phthalic anhydride, 4.9g sodium acetate and 3.0g of phenylacetic acid were heated at 230-240° C. The reaction mixture was treated as described in Examples 4 and 5, yielding 1.4g of (6), m.p. 139-140° C, and 2.8g of (7), m.p. 147-148° C.

| | | | | |
|---|---|---|---|---|
| Analysis | Calculated | : | C 63.4; | H 2.9% |
| | Found (6) | : | C 62.9; | H 2.9% |
| | Found (7) | : | C 63.1; | H 2.9% |

Example 8

Preparation of 5-(2-chloro-4-trifluoromethylphenoxy)-3-dimethylaminomethylene-1-(3H)isobenzofuranone, Z-isomer.

A mixture of 3.3g 5-(2-chloro-4-trifluoromethylphenoxy) phthalide and a catalytic amount of potassium tert-butoxide in 5ml of dimethylformamide dimethylacetal, was refluxed under nitrogen. After 1 hour and 3 hours an extra 5ml of the acetal was added to the mixture. After reflux for a further hour, the mixture was cooled, yielding crystals. The mixture was taken up in a two phase water/methylene chloride system, the organic layer was dried, the solvent was removed and the residue was chromatographically purified over silica using methylene chloride as eluant, yielding the title compound, m.p. 145-147° C.

| | |
|---|---|
| Analysis Calculated | : C 56.3; H 3.4; N 3.65% |
| Found | : C 56.1; H 3.5; N 3.0% |

Herbicidal Activity

To evaluate their herbicidal activity, the compounds prepared were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris - (SB) and soya bean, Glycine max (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in the following Table, in which the compounds are identified by reference to the preceding examples.

TABLE

| Compound of Ex. No. | Soil drench 10kg/ha Mz | R | BG | O | L | M | SB | S | Dosage kg/ha | Foliar Spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 1 | 8 | 7 | 8 | 9 | 6 | 8 | 8 | 0 | 5 | 6 | 8 | 9 | 9 | 9 | 9 | 9 | 9 | 3 | 1 | 9 | 8 | 9 | 9 | 9 | 8 |
| | | | | | | | | | 1 | 4 | 7 | 9 | 9 | 9 | 9 | 8 | 7 | 3 | 1 | 6 | 7 | 8 | 8 | 8 | 7 |
| 2 | 6 | 6 | 6 | 6 | 6 | 3 | 6 | 2 | 5 | 6 | 5 | 9 | 9 | 9 | 9 | 9 | 9 | 1 | 1 | 9 | 5 | 6 | 7 | 8 | 3 |
| | | | | | | | | | 1 | 4 | 4 | 9 | 8 | 9 | 9 | 9 | 9 | 1 | 1 | 7 | 1 | 5 | 6 | 6 | 0 |
| 3 | 0 | 0 | 0 | 0 | 0 | 0 | 0 | 0 | 5 | 4 | 2 | 6 | 2 | 5 | 4 | 5 | 4 | 0 | 0 | 0 | 0 | 0 | 0 | 0 | 0 |
| | | | | | | | | | 1 | 2 | 2 | 6 | | 5 | | | 4 | 0 | 0 | 0 | 0 | 0 | 0 | 0 | 0 |
| 4 | 0 | 0 | 0 | 0 | 0 | 0 | 0 | 0 | 5 | 3 | 0 | 3 | 2 | 5 | 4 | 3 | 3 | 0 | 0 | 0 | 0 | 0 | 0 | 0 | 0 |
| | | | | | | | | | 1 | 0 | 0 | 2 | 1 | 2 | 3 | 3 | 2 | 0 | 0 | 0 | 0 | 0 | 0 | 0 | 0 |
| 5 | 0 | 0 | 0 | 0 | 0 | 0 | 0 | 0 | 5 | 0 | 0 | 0 | 0 | 0 | 0 | 0 | 0 | 0 | 0 | 0 | 0 | 0 | 0 | 0 | 0 |
| | | | | | | | | | 1 | 0 | 0 | 0 | 0 | 0 | 0 | 0 | 0 | 0 | 0 | 0 | 0 | 0 | 0 | 0 | 0 |

TABLE

| Compound of Ex. No. | Soil drench 10kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 6 | 0 | 0 | 0 | 0 | 0 | 0 | 0 | 0 | 5 | 3 | 0 | 5 | 2 | 5 | 2 | 0 | 3 | 0 | 0 | 0 | 0 | 0 | 0 | 0 | 0 |
| | | | | | | | | | 1 | 1 | 0 | 1 | 1 | 2 | 0 | 0 | 2 | 0 | 0 | 0 | 0 | 0 | 0 | 0 | 0 |
| 7 | 0 | 0 | 0 | 0 | 0 | 0 | 0 | 0 | 5 | 0 | 0 | 0 | 0 | 0 | 0 | 0 | 0 | 0 | 0 | 0 | 0 | 0 | 0 | 0 | 0 |
| | | | | | | | | | 1 | 0 | 0 | 0 | 0 | 0 | 0 | 0 | 0 | 0 | 0 | 0 | 0 | 0 | 0 | 0 | 0 |
| 8 | 4 | 3 | 6 | 5 | 6 | 7 | 5 | 4 | 5 | 5 | 4 | 7 | 5 | 8 | 7 | 7 | 6 | 0 | 0 | 4 | 2 | 5 | 5 | 6 | 0 |
| | | | | | | | | | 1 | 3 | 2 | 5 | 5 | 7 | 6 | 6 | 5 | 0 | 0 | 2 | 0 | 3 | 2 | 4 | 0 |

## Claims

1. Diphenyl ether derivatives having the general formula I,

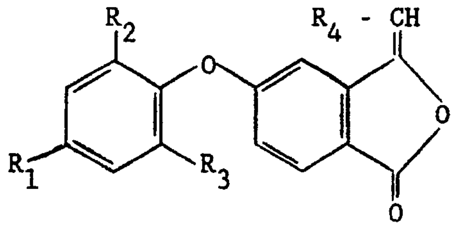

I

in which $R_1$ represents a hydrogen or halogen atom, or an alkyl or haloalkyl group; $R_2$ and $R_3$ which may be the same or different, each independently represents a hydrogen or halogen atom or an alkyl, haloalkyl, nitro or cyano group; and $R^4$ represents a hydrogen atom or an alkyl group.

2. Diphenyl ether derivatives according to claim 1, wherein $R_1$ represents a haloalkyl group, $R_2$ represents a halogen atom and $R_3$ represents a hydrogen atom.

3. Diphenyl ether derivatives according to claim 1 or 2, in which $R_4$ represents a hydrogen atom or a $C_{1-4}$ alkyl group.

4. Process for the preparation of a diphenylether derivative of formula I as defined in any one of claims 1 to 3, which comprises reacting a phthalide of formula II

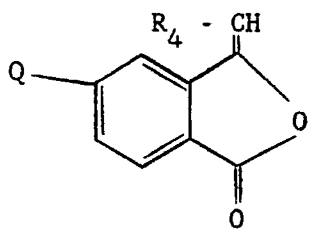

II

with a compound of the formula III

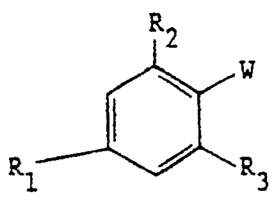

III

wherein $R_1$, $R_2$, $R_3$ and $R_4$ have the meanings as defined in any one of claims 1 to 3, one of Q and W represents a halogen atom or a nitro group and the other of W and Q represents a group of formula -OM, wherein M represents a hydrogen or alkali metal atom.

5. Process according to claim 4, in which the phthalide of formula II, wherein Q is hydroxyl, is reacted with an alkali metal hydroxide or hydride forming an alkali metal salt, and the alkali metal salt is reacted with the compound of formula III, wherein W represents a chlorine atom.

6. Process for the preparation of diphenyl ether derivatives of the formula I as defined in claim 1, which process comprises reacting a compound of formula IV

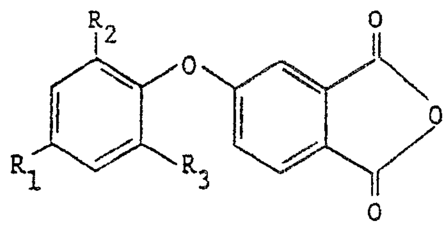

IV

with a compound of the formula $(R_4)H_2C$-L in which L is a suitable leaving group.

7. Process according to claim 6, in which L represents a carboxyl or an alkyl ester or alkali metal derivative thereof.

8. Process for the preparation of a diphenyl ether derivative of formula I, as defined in any one of claims 1 to 3, in which $R_4$ represents a hydrogen atom, which comprises dehydrating a pre-cursor compound having a >$CH$-$CH_2OH$ substituent.

9. Process for the preparation of diphenyl ether derivatives of formula I, as defined in any one of claims 1 to 3, which comprises reacting a phthalide of formula VII

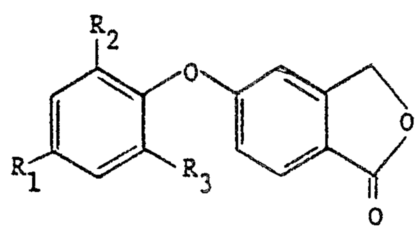

VII

wherein $R_1$, $R_2$ and $R_3$ have the meanings as defined in any one of claims 1 to 3, with an acetal of the formula

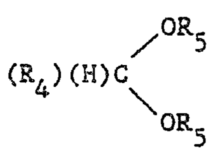

in which each $R_5$ individually represents an alkyl group.

10. Herbicidal composition which comprises a diphenyl ether derivative as claimed in any one of claims 1 to 3, together with a carrier.

11. Composition according to claim 10, which comprises at least two carriers, at least one of which is a surface-active agent.

12. Method of combating undesired plant growth at a locus, which comprises treating the locus with a diphenyl ether derivative as claimed in any one of claims 1 to 3, or with a composition as claimed in claim 10 or 11.

13. Use of diphenyl ether derivative according to any one of claims 1 to 3, as a herbicide.

Claims for the following Contracting State : AT

1. Herbicidal composition which comprises a compound having the general formula I,

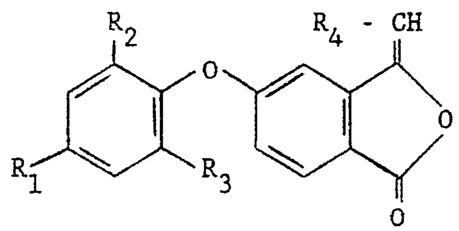

I

in which $R_1$ represents a hydrogen or halogen atom, or an alkyl or haloalkyl group; $R_2$ and $R_3$ which may be the same or different, each independently represents a hydrogen or halogen atom or an alkyl, haloalkyl, nitro or cyano group; and $R^4$ represents a hydrogen atom or an alkyl group, together with a carrier.

2. A composition according to claim 1, wherein, in the general formula I, $R_1$ represents a haloalkyl group, $R_2$ represents a halogen atom and $R_3$ represents a hydrogen atom.

3. A composition according to claim 1 or 2, wherein, in the general formula I, $R_4$ represents a hydrogen atom or a $C_{1-4}$ alkyl group.

4. A composition according to any one of claims 1 to 3, which comprises at least two carriers, at least one of which is a surface-active agent.

5. Process for the preparation of a diphenyl ether derivative of formula I as defined in any one of claims 1 to 3, which comprises reacting a phthalide of formula II

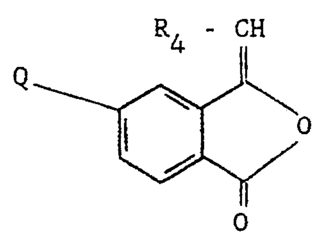

II

with a compound of the formula III

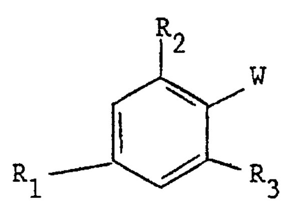

III

wherein $R_1$, $R_2$, $R_3$ and $R_4$ have the meanings as defined in any one of claims 1 to 3, one of Q and W represents a halogen atom or a nitro group and the other of W and Q represents a group of formula -OM, wherein M represents a hydrogen or alkali metal atom.

6. Process according to claim 5, in which the phthalide of formula II, wherein Q is hydroxyl, is reacted with an alkali metal hydroxide or hydride forming an alkali metal salt, and the alkali metal salt is reacted with the compound of formula III, wherein W represents a chlorine atom.

7. Process for the preparation of diphenyl ether derivatives of the formula I as defined in claim 1, which process comprises reacting a compound of formula IV

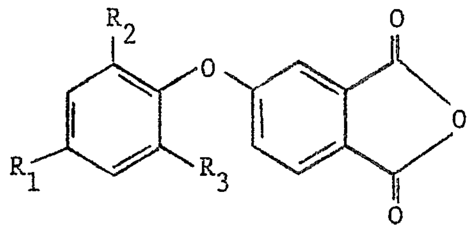

IV

with a compound of the formula $(R_4)H_2C$-L in which L is a suitable leaving group.

**8.** Process according to claim 7, in which L represents a carboxyl or an alkyl ester or alkali metal derivative thereof.

**9.** Process for the preparation of a diphenyl ether derivative of formula I, as defined in any one of claims 1 to 3, in which $R_4$ represents a hydrogen atom, which comprises dehydrating a pre-cursor compound having a $>CH-CH_2OH$ substituent.

**10.** Process for the preparation of diphenyl ether derivatives of formula I, as defined in any one of claims 1 to 3, which comprises reacting a phthalide of formula VII

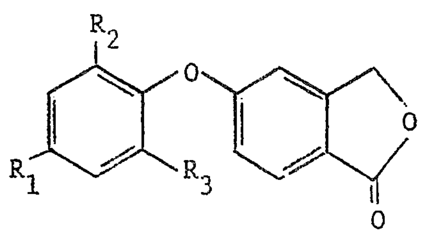

VII

wherein $R_1$, $R_2$ and $R_3$ have the meanings as defined in any one of claims 1 to 3, with an acetal of the formula

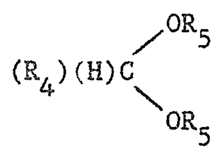

in which each $R_5$ individually represents an alkyl group.

**11.** Method of combating undesired plant growth at a locus, which comprises treating the locus with a diphenyl ether derivative as defined in any one of claims 1 to 3, or with a composition as claimed in any one of claims 1 to 4.

**12.** Use of diphenyl ether derivative as defined in any one of claims 1 to 3, as a herbicide.

Claims for the following Contracting State : ES

**1.** Process for the preparation of a diphenyl ether derivative of the general formula I

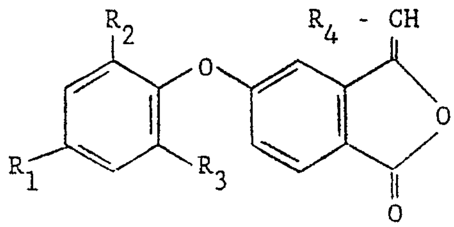

I

in which $R_1$ represents a hydrogen or halogen atom, or an alkyl or haloalkyl group;
$R_2$ and $R_3$ which may be the same or different, each independently represents a hydrogen or halogen atom or an alkyl, haloalkyl, nitro or cyano group; and $R^4$ represents a hydrogen atom or an alkyl group, which comprises reacting a phthalide of formula II

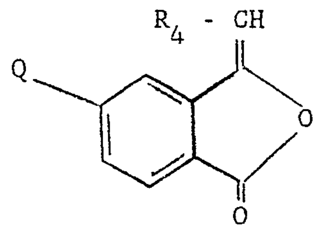

II

with a compound of the formula III

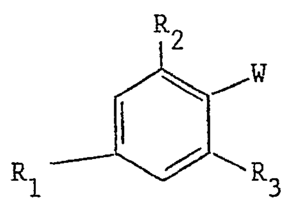

III

wherein $R_1$, $R_2$, $R_3$ and $R_4$ have the meanings given above, one of Q and W represents a halogen atom or a nitro group and the other of W and Q represents a group of formula -OM, wherein M represents a hydrogen or alkali metal atom.

2. Process according to claim 1, in which the phthalide of formula II, wherein Q is hydroxyl, is reacted with an alkali metal hydroxide or hydride forming an alkali metal salt, and the alkali metal salt is reacted with the compound of formula III, wherein W represents a chlorine atom.

3. Process for the preparation of diphenyl ether derivatives of the formula I as defined in claim 1, which process comprises reacting a compound of formula IV

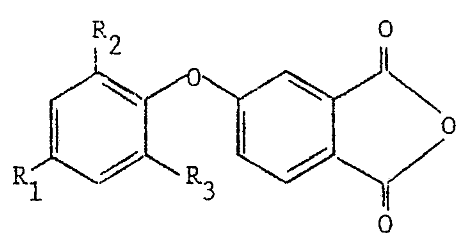

IV

with a compound of the formula $(R_4)H_2C\text{-}L$ in which L is a suitable leaving group.

4. Process according to claim 3, in which L represents a carboxyl or an alkyl ester or alkali metal derivative thereof.

5. Process for the preparation of a diphenyl ether derivative of formula I, as defined in claim 1, in which $R_4$ represents a hydrogen atom, which comprises dehydrating a pre-cursor compound having a >CH-$CH_2OH$ substituent.

6. Process for the preparation of diphenyl ether derivatives of formula I, as defined in claim 1, which comprises reacting a phthalide of formula VII

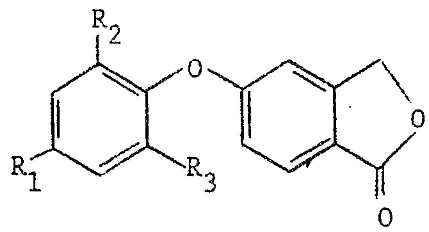

VII

wherein $R_1$, $R_2$ and $R_3$ have the meanings as defined in claim 1, with an acetal of the formula

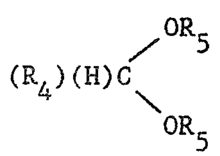

in which each $R_5$ individually represents an alkyl group.

7. Process according to any one of claims 1 to 6, wherein a compound is prepared in which $R_1$ represents a haloalkyl group, $R_2$ represents a halogen atom and $R_3$ represents a hydrogen atom.

8. Process according to any one of claims 1 to 7, wherein a compound is prepared in which $R_4$ represents a hydrogen atom or a $C_{1-4}$ alkyl group.

9. Process for the preparation of a herbicidal composition which comprises bringing a diphenyl ether derivative prepared by a process as claimed in any one of claims 1 to 8, into association with at least one carrier.

10. Process according to claim 9, wherein a composition is prepared which comprises at least two carriers, at least one of which is a surface-active agent.

11. Process for combating undesired plant growth at a locus, which comprises treating the locus with a diphenyl ether derivative prepared by a process as claimed in any one of claims 1 to 8, or with a composition prepared by a process as claimed in claim 9 or 10.

## Revendications

1. Dérivés d'éthers diphényliques ayant la formule générale I

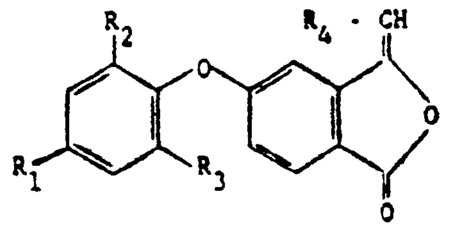

I

où $R_1$ représente un atome d'hydrogène ou d'un halogène ou un groupe alcoyle ou haloalcoyle ; $R_2$ et $R_3$, qui peuvent être identiques ou différents, représentent chacun indépendamment un atome d'hydrogène ou d'halogène ou un groupe alcoyle, haloalcoyle, nitro ou cyano ; et $R_4$ représente un atome d'hydrogène ou un groupe alcoyle.

2. Dérivés d'éthers diphényliques selon la revendication 1, où $R_1$ représente un groupe haloalcoyle, $R_2$ représente un atome d'halogène et $R_3$ représente un atome d'hydrogène.

3. Dérivés d'éthers diphényliques selon la revendication 1 ou 2, dans lesquels $R_4$ représente un atome d'hydrogène ou un groupe alcoyle en $C_{1-4}$.

4. Procédé de préparation d'un dérivé d'éther diphénylique de formule I tel que défini dans l'une quelconque des revendications 1 à 3, qui comprend la réaction d'un phtalide de formule II

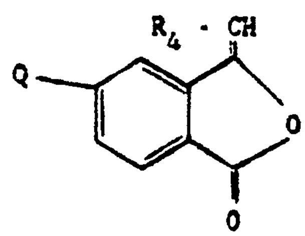

II

avec un composé de formule III

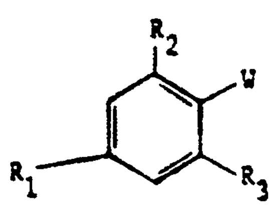

III

où $R_1$, $R_2$, $R_3$ et $R_4$ ont les significations définies dans l'une quelconque des revendications 1 à 3, un de Q et W représente un atome d'hydrogène ou un groupe nitro et l'autre représente un groupe de formule -OM, où M représente un atome d'hydrogène ou d'un métal alcalin.

5. Procédé selon la revendication 4, dans lequel le phtalide de formule II, où Q est un groupe hydroxyle, est mis à réagir avec un hydroxyde ou hydrure de métal alcalin pour former un sel de métal alcalin et le sel de métal alcalin est mis à réagir avec le composé de formule III, où W représente un atome de chlore.

6. Procédé de préparation de dérivés d'éthers diphényliques de la formule I tels que définis dans la revendication 1, qui comprend la réaction d'un composé de formule IV

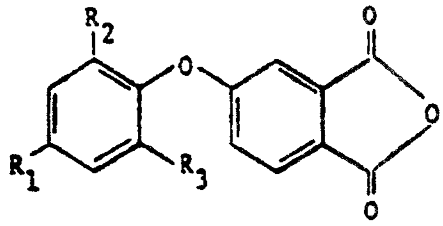

IV

avec un composé de la formule $(R_4)H_2C$-L où L est un groupe qui part approprié.

7. Procédé selon la revendication 6, dans lequel L représente un groupe carboxyle ou un ester d'alcoyle ou dérivé de métal alcalin correspondant.

8. Procédé de préparation d'un dérivé d'éther diphénylique de formule I, tel que défini dans l'une quelconsue des revendications 1 à 3, qui comprend la déshydratation d'un composé progéniteur ayant un substituant $>CH-CH_2OH$.

9. Procédé de préparation de dérivés d'éthers diphényliques de formule I tels que définis dans l'une quelconque des revendications 1 à 3, qui comprend la réaction d'un phtalide de formule VII

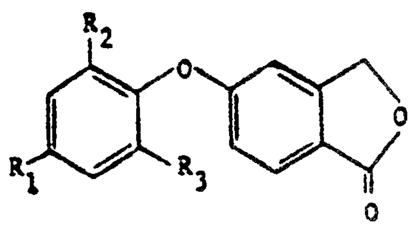

VII

où $R_1$, $R_2$ et $R_3$ ont les significations définies dans l'une quelconque des revendications 1 à 3, avec un acétal de la formule

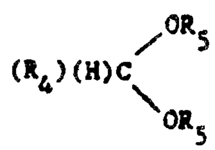

où chaque $R_5$ individuellement représente un groupe alcoyle.

10. Composition herbicide qui comprend un dérivé d'éther diphénylique selon l'une quelconque des revendications 1 à 3 en même temps qu'un véhicule.

11. Composition selon la revendication 10, qui comprend au moins deux véhicules, dont au moins un est un agent tensio-actif.

12. Méthode de lutte contre la croisaance de plantes indésirables en un lieu, qui comprend le traitement du lieu avec un dérivé d'éther diphénylique selon l'une quelconque des revendications 1 à 3 ou avec une composition selon la revendication 10 ou 11.

13. Utilisation d'un dérivé d'éther diphénylique selon l'une quelconque des revendications 1 à 3 comme herbicide.

Revendications pour l'Etat contractant suivant : AT

1. Composition herbicide qui comprend un composé ayant la formule générale I

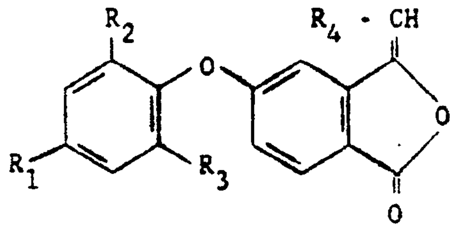

I

où $R_1$ représente un atome d'hydrogène ou d'un halogène ou un groupe alcoyle ou haloalcoyle ; $R_2$ et $R_3$, qui peuvent être identiques ou différents, représentent chacun indépendamment un atome d'hydrogène ou d'halogène ou un groupe alcoyle, haloalcoyle, nitro ou cyano ; et $R_4$ représente un atome d'hydrogène ou un groupe alcoyle ; en même temps qu'un véhicule.

2. Une composition selon la revendication 1, dans laquelle, dans la formule générale I, $R_1$ représente un groupe haloalcoyle, $R_2$ représente un atome d'halogène et $R_3$ représente un atome d'hydrogène.

3. Une composition selon la revendication 1 ou 2, dans laquelle, dans la formule générale I, $R_4$ représente un atome d'hydrogène ou un groupe alcoyle en $C_{1-4}$.

4. Une composition selon l'une quelconque des revendications 1 à 3, qui comprend au moins deux véhicules, dont au moins un est un agent tensio-actif.

5. Procédé de préparation d'un dérivé d'éther diphénylique de formule I tel que défini dans l'une quelconque des revendications 1 à 3, qui comprend la réaction d'un phtalide de formule II

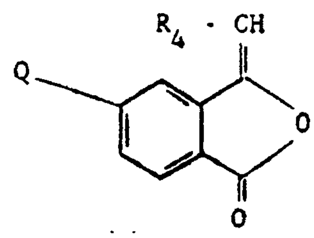

II

avec un composé de formule III

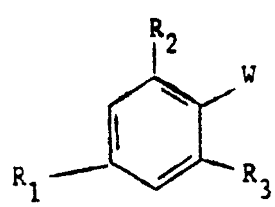

III

où $R_1$, $R_2$, $R_3$ et $R_4$ ont les significations définies dans l'une quelconque des revendications 1 à 3, un de Q et W représente un atome d'halogène ou un groupe nitro et l'autre représente un groupe de formule -OM, où M représente un atome d'hydrogène ou d'un métal alcalin.

6. Procédé selon la revendication 5, dans lequel le phtalide de formule II, où Q est un groupe hydroxyle, est mis à réagir avec un hydroxyde ou hydrure de métal alcalin pour former un sel de métal alcalin et le sel de métal alcalin est mis à réagir avec le composé de formule III, où W représente un atome de chlore.

7. Procédé de préparation de dérivés d'éthers diphényliques de la formule I tels que définis dans la revendication 1, qui comprend la réaction d'un composé de formule IV

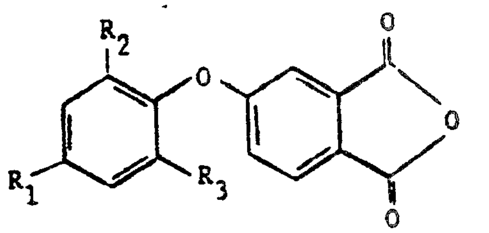

IV

avec un composé de la formule $(R_4)H_2C$-L, où L est un groupe qui part approprié.

8. Procédé selon la revendication 7, dans lequel L représente un groupe carboxyle ou un ester d'alcoyle ou dérivé se métal alcalin correspondant.

9. Procédé de préparation d'un dérivé d'éther diphénylique de formule I tel que défini dans l'une quelconque des revendications 1 à 3, où $R_4$ représente un atome d'hydrogène, qui comprend la déshydratation d'un composé progéniteur ayant un substituant >CH-$CH_2$OH.

10. Procédé de préparation de dérivés d'éthers diphényliques de formule I tels que définis dans l'une quelconque des revendications 1 à 3, qui comprend la réaction d'un phtalide de formule VII

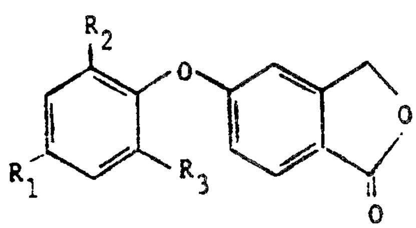

VII

où $R_1$, $R_2$ et $R_3$ ont les significations définies dans l'une quelconque des revendications 1 à 3, avec un acétal de la formule

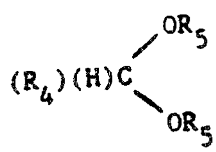

où chaque $R_5$ individuellement représente un groupe alcoyle.

11. Méthode de lutte contre la croissance de plantes indésirables en un lieu, qui comprend le traitement du lieu avec un dérivé d'éther diphénylique tel que défini dans l'une quelconque des revendications 1 à 3 ou avec une composition selon l'une quelconque des revendications 1 à 4.

12. Utilisation d'un dérivé d'éther diphénylique tel que défini dans l'une quelconque des revendications 1 à 3 comme herbicide.

Revendications pour l'Etat contractant suivant : ES

1. Procédé de préparation d'un dérivé d'éther diphénylique de la formule générale I

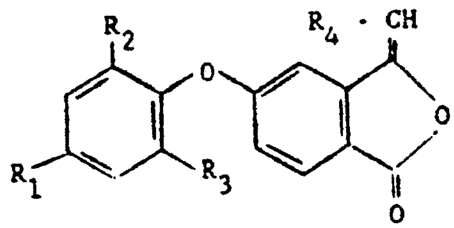

I

où $R_1$ représente un atome d'hydrogène ou d'un halogène ou un groupe alcoyle ou haloalcoyle ; $R_2$ et $R_3$, qui peuvent être identiques ou différents, représentent chacun indépendamment un atome d'hydrogène ou d'halogène ou un groupe alcoyle, haloalcoyle, nitro ou cyano ; et $R^4$ représente un atome d'hydrogène ou un groupe alcoyle, qui comprend la réaction d'un phtalide de formule II

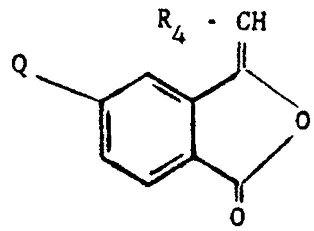

II

avec un composé de formule III

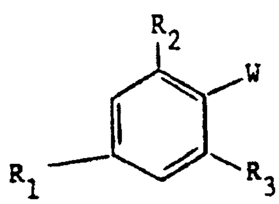

III

où $R_1$, $R_2$, $R_3$ et $R_4$ ont les significations indiquées ci-dessus, un de Q et W représente un atome d'halogène ou un groupe nitro et l'autre représente un groupe de formule -OM, où M représente un atome d'hydrogène ou d'un métal alcalin.

**2.** Procédé selon la revendication 1, dans lequel le phtalide de formule II, où Q est un groupe hydroxyle, est mis à réagir avec un hydroxyde ou hydrure de métal alcalin pour former un sel de métal alcalin, et le sel de métal alcalin est mis à réagir avec un composé de formule III, où W représente un atome de chlore.

**3.** Procédé de préparation de dérivés d'éthers diphényliques de la formule I tels que définis dans la revendication 1, qui comprend la réaction d'un composé de formule IV

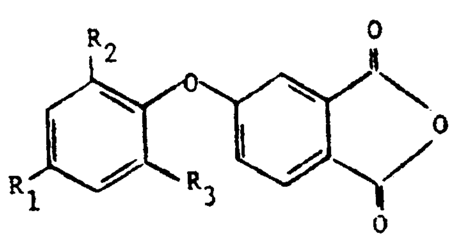

IV

avec un composé de la formule $(R_4)H_2C$-L, où L est un groupe qui part approprié.

4. Procédé selon la revendication 3, dans lequel L représente un groupe carboxyle ou un ester d'alcoyle ou dérivé de métal alcalin correspondant.

5. Procédé de préparation d'un dérivé d'éther diphénylique de formule I tel que défini dans la revendication 1, où $R_4$ représente un atome d'hydrogène, qui comprend la déshydratation d'un composé progéniteur ayant un substituant $>CH-CH_2OH$.

6. Procédé de préparation de dérivés d'éthers diphényliques de formule I tels que définis dans la revendication 1, qui comprend la réaction d'un phtalide de formule VII

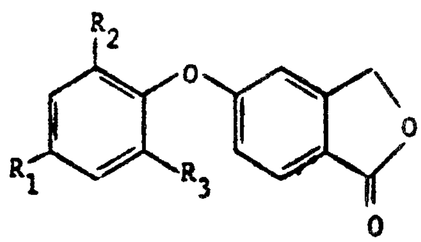

VII

où $R_1$, $R_2$ et $R_3$ ont les significations définies dans la revendication 1, avec un acétal de la formule

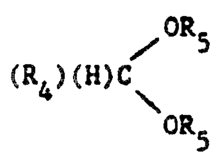

où chaque $R_5$ individuellement représente un groupe alcoyle.

7. Procédé selon l'une quelconque des revendications 1 à 6, dans lequel on prépare un composé dans lequel $R_1$ représente un groupe haloalcoyle, $R_2$ représente un atome d'halogène et $R_3$ représente un atome d'hydrogène.

8. Procédé selon l'une quelconque des revendications 1 à 7, dans lequel on prépare un composé dans lequel $R_4$ représente un atome d'hydrogène ou un groupe alcoyle en $C_{1-4}$.

9. Procédé de préparation d'une composition herbicide, selon lequel on met un dérivé d'éther diphénylique préparé par un procédé selon l'une quelconque des revendications 1 à 8 en association avec au moins un véhicule.

10. Procédé selon la revendication 9, dans lequel on prépare une composition qui comprend au moins deux véhicules, dont au moins un est un agent tensio-actif.

11. Procédé de lutte contre la croissance de plantes indésirables en un lieu, qui comprend le traitement du lieu avec un dérivé d'éther diphénylique préparé par un procédé selon l'une quelconque des revendications 1 à 8 ou avec une composition préparée par un procédé selon la revendication 9 ou 10.

## Ansprüche

1. Diphenyletherderivate mit der allgemeinen Formel I:

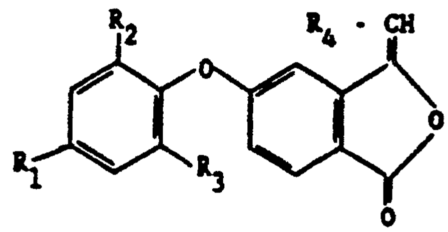

I,

worin

- $R_1$ ein Wasserstoff- oder Halogenatom oder eine Alkyl- oder Halogenalkylgruppe darstellt;
- $R_2$ und $R_3$, die gleich oder verschieden sein können, jeweils unabhängig voneinander ein Wasserstoff- oder Halogenatom oder eine Alkyl-, Halogenalkyl-, Nitro- oder Cyanogruppe bedeuten; und
- $R^4$ ein Wasserstoffatom oder eine Alkylgruppe darstellt.

**2.** Diphenyletherderivate nach Anspruch 1, worin

- $R_1$ eine Halogenalkylgruppe darstellt,
- $R_2$ ein Halogenatom bedeutet und
- $R_3$ ein Wasserstoffatom darstellt.

**3.** Diphenyletherderivate nach Anspruch 1 oder 2, worin $R_4$ ein Wasserstoffatom oder eine $C_{1-4}$Alkylgruppe bedeutet.

**4.** Verfahren zur Herstellung eines Diphenyletherderivats der Formel I, wie in einem der Ansprüche 1 bis 3 definiert, welches ein Umsetzen eines Phthalids der Formel II

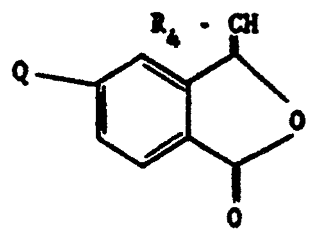

II

mit einer Verbindung der Formel III

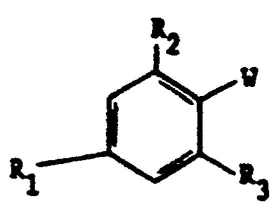

III,

worin $R_1$, $R_2$, $R_3$ und $R_4$ die in einem der Ansprüche 1 bis 3 definierten Bedeutungen besitzen und einer der Reste Q und W ein Halogenatom oder eine Nitrogruppe bedeutet und der andere Rest von W und Q eine Gruppe der Formel -OM bezeichnet, worin M ein Wasserstoffatom oder ein Alkalimetallatom darstellt, umfaßt.

**5.** Verfahren nach Anspruch 4, worin das Phthalid der Formel II, in welcher Q für Hydroxyl steht, mit einem Alkalimetallhydroxid oder -hydrid unter Ausbildung eines Alkalimetallsalzes umgesetzt wird und das Alkalimetallsalz mit der Verbindung der Formel III, worin W ein Chloratom darstellt, umgesetzt wird.

6. Verfahren zur Herstellung von Diphenyletherderivaten der Formel I, wie in Anspruch 1 definiert, welches Verfahren ein Umsetzen einer Verbindung der Formel IV

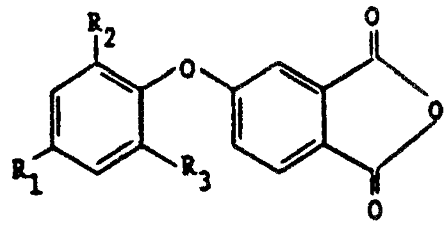

IV

mit einer Verbindung der Formel $(R_4)H_2C$-L, worin L eine geeignete Leaving-Gruppe darstellt, umfaßt.

7. Verfahren nach Anspruch 6, worin L eine Carboxylgruppe oder ein Alkylester- oder Alkalimetallderviat hievon darstellt.

8. Verfahren zur Herstellung eines Diphenyletherderivats der Formel I, wie in einem der Ansprüche 1 bis 3 definiert, worin $R_4$ ein Wasserstoffatom bedeutet, welches Verfahren ein Dehydratisieren einer Vorläuferverbindung umfaßt, die einen $>CH-CH_2OH$-Substituenten trägt.

9. Verfahren zur Herstellung von Diphenyletherderivaten der Formel I, wie in einem der Ansprüche 1 bis 3 definiert, welches ein Umsetzen eines Phthalids der Formel VII

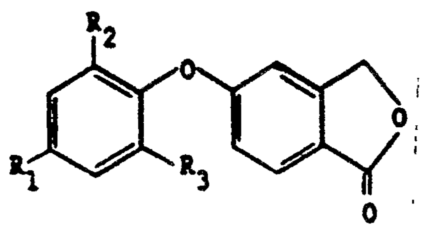

. VII,

worin $R_1$, $R_2$ und $R_3$ die in einem der Ansprüche 1 bis 3 angegebene Bedeutung haben, mit einem Acetal der Formel

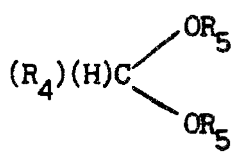

,

worin jeder Rest $R_5$ unabhängig voneinander eine Alkylgruppe bedeutet, umfaßt.

10. Herbizide Zusammensetzung, welche ein Diphenyletherderivat, wie in einem der Ansprüche 1 bis 3 beansprucht, zusammen mit einem Träger umfaßt.

11. Zusammensetzung nach Anspruch 10, welche wenigstens zwei Träger umfaßt, von denen wenigstens einer ein oberflächenaktives Mittel ist.

12. Verfahren zur Bekämpfung von unerwünschtem Pflanzenwuchs an einem Ort, welches ein Behandeln des Ortes mit einem Diphenyletherderivat, wie in einem der Ansprüche 1 bis 3 beansprucht, oder mit einer Zusammensetzung, wie in Anspruch 10 oder 11 beansprucht, umfaßt.

13. Verwendung eines Diphenyletherderivats nach einem der Ansprüche 1 bis 3 als ein Herbizid.

Patentansprüche für folgenden Vertragsstaat : AT

1. Herbizide Zusammensetzung, welche eine Verbindung mit der allgemeinen Formel I:

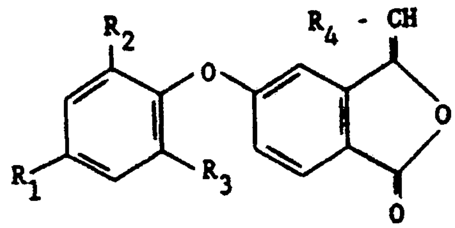

worin

$R_1$ ein Wasserstoff- oder Halogenatom oder eine Alkyl- oder Halogenalkylgruppe darstellt;

$R_2$ und $R_3$, die gleich oder verschieden sein können, jeweils unabhängig voneinander ein Wasserstoff- oder Halogenatom oder eine Alkyl-, Halogenalkyl-, Nitro- oder Cyanogruppe bedeuten; und

$R^4$ ein Wasserstoffatom oder eine Alkylgruppe darstellt, zusammen mit einem Träger umfaßt.

2. Zusammensetzung nach Anspruch 1, worin in der allgemeinen Formel I

$R_1$ eine Halogenalkylgruppe,

$R_2$ ein Halogenatom und

$R_3$ ein Wasserstoffatom bedeutet.

3. Zusammensetzung nach Anspruch 1 oder 2, worin in der allgemeinen Formel I $R_4$ ein Wasserstoffatom oder eine $C_{1-4}$Alkylgruppe bedeutet.

4. Zusammensetzung nach einem der Ansprüche 1 bis 3, welche wenigstens zwei Träger umfaßt, von denen wenigstens einer ein oberflächenaktives Mittel ist.

5. Verfahren zur Herstellung eines Diphenyletherderivats der Formel I, wie in einem der Ansprüche 1 bis 3 definiert, welches ein Umsetzen eines Phthalids der Formel II

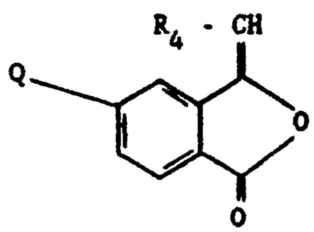

II

mit einer Verbindung der Formel III

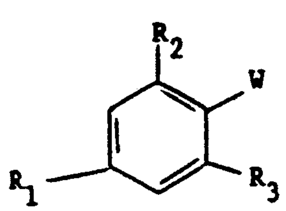

III,

worin $R_1$, $R_2$, $R_3$ und $R_4$ die in einem der Ansprüche 1 bis 3 definierten Bedeutungen besitzen und einer der Reste Q und W ein Halogenatom oder eine Nitrogruppe bedeutet und der andere Rest von W und Q eine Gruppe der Formel -OM bezeichnet, worin M ein Wasserstoffatom oder ein Alkalimetallatom darstellt, umfaßt.

6. Verfahren nach Anspruch 5, worin das Phthalid der Formel II, in welcher Q für Hydroxyl steht, mit einem Alkalimetallhydroxid oder -hydrid unter Ausbildung eines Alkalimetallsalzes umgesetzt wird und das Alkalimetallsalz mit der Verbindung der Formel III, worin W ein Chloratom darstellt, umgesetzt wird.

7. Verfahren zur Herstellung von Diphenyletherderivaten der Formel I, wie in Anspruch 1 definiert, welches Verfahren ein Umsetzen einer Verbindung der Formel IV

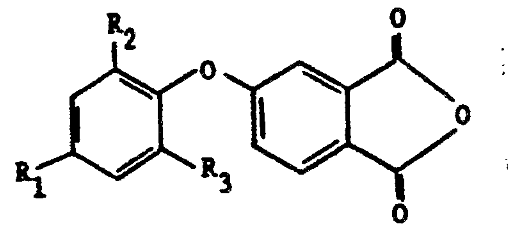

IV

mit einer Verbindung der Formel $(R_4)H_2C\text{-}L$, worin L eine geeignete Leaving-Gruppe darstellt, umfaßt.

8. Verfahren nach Anspruch 7, worin L eine Carboxylgruppe oder ein Alkylester- oder Alkalimetallderviat hievon darstellt.

9. Verfahren zur Herstellung eines Diphenyletherderivats der Formel I, wie in einem der Ansprüche 1 bis 3 definiert, worin $R_4$ ein Wasserstoffatom bedeutet, welches Verfahren ein Dehydratisieren einer Vorläuferverbindung umfaßt, die einen $>CH\text{-}CH_2OH$-Substituenten trägt.

10. Verfahren zur Herstellung von Diphenyletherderivaten der Formel I, wie in einem der Ansprüche 1 bis 3 definiert, welches ein Umsetzen eines Phthalids der Formel VII

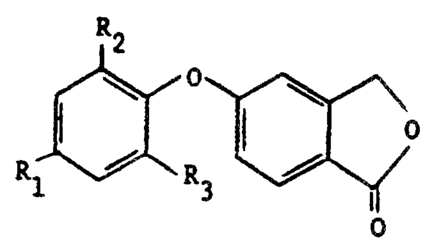

VII,

worin $R_1$, $R_2$ und $R_3$ die in einem der Ansprüche 1 bis 3 angegebene Bedeutung haben, mit einem Acetal der Formel

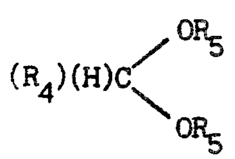

,

worin jeder Rest $R_5$ unabhängig voneinander eine Alkylgruppe bedeutet, umfaßt.

11. Verfahren zur Bekämpfung von unerwünschtem Pflanzenwuchs an einem Ort, welches ein Behandeln des Ortes mit einem Diphenyletherderivat, wie in einem der Ansprüche 1 bis 3 definiert, oder mit einer Zusammensetzung, wie in einem der Ansprüche 1 bis 4 beansprucht, umfaßt.

12. Verwendung eines Diphenyletherderivats, wie in einem der Ansprüche 1 bis 3 definiert, als ein Herbizid.

Patentansprüche für folgenden Vertragsstaat : ES

1. Verfahren zur Herstellung eines Diphenyletherderivats mit der allgemeinen Formel I:

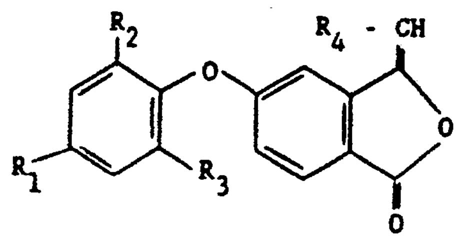

I,

worin

$R_1$ ein Wasserstoff- oder Halogenatom oder eine Alkyl- oder Halogenalkylgruppe darstellt;

$R_2$ und $R_3$, die gleich oder verschieden sein können, jeweils unabhängig voneinander ein Wasserstoff- oder Halogenatom oder eine Alkyl-, Halogenalkyl-, Nitro- oder Cyanogruppe bedeuten; und

$R^4$ ein Wasserstoffatom oder eine Alkylgruppe darstellt,

welches ein Umsetzen eines Phthalids der Formel II

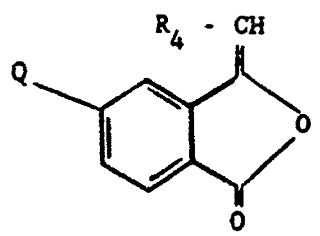

II

mit einer Verbindung der Formel III

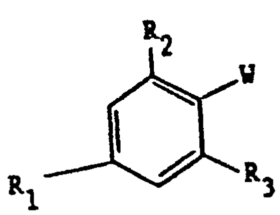

III,

worin $R_1$, $R_2$, $R_3$ und $R_4$ die oben definierten Bedeutungen besitzen und einer der Reste Q und W ein Halogenatom oder eine Nitrogruppe bedeutet und der andere Rest von W und Q eine Gruppe der Formel -OM bezeichnet, worin M ein Wasserstoffatom oder ein Alkalimetallatom darstellt, umfaßt.

2. Verfahren nach Anspruch 1, worin das Phthalid der Formel II, in welcher Q für Hydroxyl steht, mit einem Alkalimetallhydroxid oder -hydrid unter Ausbildung eines Alkalimetallsalzes umgesetzt wird und das Alkalimetallsalz mit der Verbindung der Formel III, worin W ein Chloratom darstellt, umgesetzt wird.

3. Verfahren zur Herstellung von Diphenyletherderivaten der Formel I, wie in Anspruch 1 definiert, welches Verfahren ein Umsetzen einer Verbindung der Formel IV

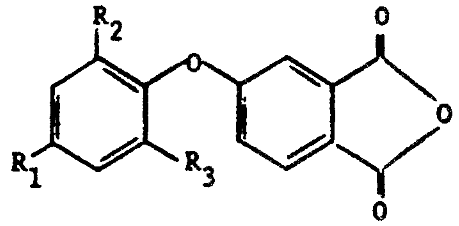

IV

mit einer Verbindung der Formel $(R_4)H_2C\text{-}L$, worin L eine geeignete Leaving-Gruppe darstellt, umfaßt.

4. Verfahren nach Anspruch 3, worin L eine Carboxylgruppe oder ein Alkylester-oder Alkalimetallderviat hievon darstellt.

5. Verfahren zur Herstellung eines Diphenyletherderivats der Formel I, wie in Anspruch 1 definiert, worin $R_4$ ein Wasserstoffatom bedeutet, welches Verfahren ein Dehydratisieren einer Vorläuferverbindung umfaßt, die einen $>CH\text{-}CH_2OH$-Substituenten trägt.

6. Verfahren zur Herstellung von Diphenyletherderivaten der Formel I, wie in Anspruch 1 definiert, welches ein Umsetzen eines Phthalids der Formel VII

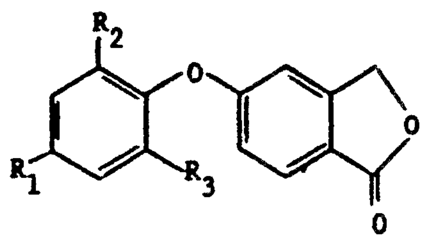

VII,

worin $R_1$, $R_2$ und $R_3$ die in Anspruch 1 angegebene Bedeutung haben, mit einem Acetal der Formel

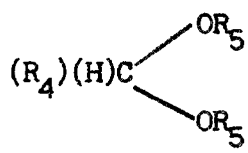

,

worin jeder Rest $R_5$ unabhängig voneinander eine Alkylgruppe bedeutet, umfaßt.

7. Verfahren nach einem der Ansprüche 1 bis 6, worin eine Verbindung hergestellt wird, in welcher
   - $R_1$ eine Halogenalkylgruppe,
   - $R_2$ ein Halogenatom und
   - $R_3$ ein Wasserstoffatom bedeutet.

8. Verfahren nach einem der Ansprüche 1 bis 7, worin eine Verbindung hergestellt wird, in welcher $R_4$ ein Wasserstoffatom oder eine $C_{1-4}$Alkylgruppe bedeutet.

9. Verfahren zur Herstellung einer herbiziden Zusammensetzung, welches ein Zusammenbringen eines Diphenyletherderivats, das nach einem Verfahren nach einem der Ansprüche 1 bis 8 hergestellt ist, mit wenigstens einem Träger umfaßt.

10. Verfahren nach Anspruch 9, worin eine Zusammensetzung bereitet wird, welche wenigstens zwei Träger umfaßt, von denen wenigstens einer ein oberflächenaktives Mittel ist.

11. Verfahren zur Bekämpfung von unerwünschtem Pflanzenwuchs an einem Ort, welches ein Behandeln des Ortes mit einem Diphenyletherderivat, hergestellt nach einem der Ansprüche 1 bis 8, oder mit einer Zusammensetzung, hergestellt nach einem Verfahren gemäß Anspruch 9 oder 10, umfaßt.